# EUROPÄISCHE PATENTSCHRIFT

(11) **EP 2 384 692 B1**
(45) Veröffentlichungstag und Bekanntmachung des Hinweises auf die Patenterteilung: **09.09.2020**
(21) Anmeldenummer: 10004872.7
(22) Anmeldetag: 07.05.2010
(51) Int. Cl.: G01B 9/02

(54) **Anordnung und Verfahren zur Interferometrie**
Method and device for interferometry
Appareil et procédé destinés à l'interférométrie

(43) Veröffentlichungstag der Anmeldung: 09.11.2011
(73) Patentinhaber: Rowiak GmbH, 30419 Hannover (DE)
(72) Erfinder: Lubatschowski, Holger, 30989 Gehrden (DE); Massow, Ole, 30159 Hannover (DE)
(74) Vertreter: Grünecker Patent- und Rechtsanwälte PartG mbB

(56) Entgegenhaltungen:
- WO-A1-01/38820
- WO-A1-2009/149103
- US-A1- 2001 000 978
- US-A1- 2006 062 260
- US-A1- 2006 139 656
- US-A1- 2007 081 166
- US-A1- 2007 206 197
- US-A1- 2008 285 043

## Beschreibung

Die vorliegende Erfindung bezieht sich auf eine Anordnung zur Interferometrie gemäß dem Oberbegriff des Anspruchs 1, sowie auf ein entsprechendes Verfahren.

In der Medizintechnik, bei der Untersuchung von biologischen Proben und in der Materialkunde haben sich interferometrische Messverfahren mittlerweile etabliert. Sie erlauben auf nicht-invasive Weise das Erlangen von Informationen über den inneren Aufbau der jeweiligen Probe. Zu diesem Zweck wird generell die aus einer bestimmten Tiefe innerhalb einer Probe zurückreflektierte Strahlung mit einem dazu kohärenten Strahlungsanteil zur Interferenz gebracht, der zuvor einen Referenzarm bestimmter Länge durchlaufen hat. In der Medizintechnik wird dieses Messverfahren als optische Kohärenztomographie (englisch: Optical Coherence Tomography, OCT) bezeichnet. Grundsätzlich unterscheidet man dabei zwischen der Zeitbereich-OCT (time domain OCT, TD-OCT) und der Frequenzbereich-OCT (frequency domain OCT, FD-OCT). Bei der TD-OCT wird der Referenzarm in seiner Länge verändert und kontinuierlich die Intensität der Interferenz gemessen, um die Materialeigenschaften der Probe in unterschiedlichen Tiefen zu messen. Bei der FD-OCT wird die Interferenz der einzelnen spektralen Komponenten des Signals erfasst. Dies hat den Vorteil, dass der Referenzarm des Interferometers nicht mehr in seiner Länge verändert werden muss.

Die FD-OCT-Geräte verwenden als Detektor ein Spektrometer. Gemessen wird die sog. Spektrale Interferenz, eine Modulation des Spektrums, wobei die Modulationsfrequenz proportional zu dem Weglängenunterschied von Referenzspiegel zu einem Objekt im Probenarm ist. Da sich so die Frequenzen verschiedener Objekte superpositionieren lassen, kann mit diesem Verfahren die gesamte Tiefeninformation mit einer einzigen Messung erfasst werden.

Herkömmliche optische Dual-Beam-(OCT-)Anordnungen und entsprechende Verfahren zur interferometrie sind beispielsweise bekannt aus A. Baumgartner, C.K. Hitzenberger, Ergun, M. Stur, H. Sattmann, W. Drexler, A.F. Fercher, "Resolution-improved dual-beam and standard optical coherence tomography: A comparison", Graefe's Arch. Clin. Exp. Ophthalmology 238, 385-392 (2000), aus W. Drexler, C.K. Hitzenberger, H. Sattmann, A.F. Fercher, "Measurement of the thickness of fundus layers by partial coherence tomography", Opt. Eng. 34, 701-710 (1995), aus W. Drexler, O. Findl, R. Menapace, A. Kruger, A. Wedrich, G. Rainer, A. Baumgartner, C.K. Hitzenberger, , A.F. Fercher, "Dual beam optical coherence tomography: signal identification for Ophthalmologie diagnosis", Journal of Biomedical Optics 3(1), 55-65 (1998), aus A.F. Fercher, W. Drexler, C.K. Hitzenberger, T. Lasser, "Optical coherence tomography - Principles and applications", Rep. Prog. Phys. 66, 239-303 (2003), oder aus C.K. Hitzenberger, "Optical Measurement of the axial eye length by laser Doppler interferometry", Investigative Ophthalmology & Visual Science 32(3), 616-624 (1991).

Im medizinischen Bereich wird die OCT in sehr geringem Maße zur Krebsdiagnose und zur Hautuntersuchung verwendet, in größerem Umfang jedoch im Bereich der Augenheilkunde. Gegenüber anderem Gewebe bietet das Auge den Vorteil, dass seine Komponenten für die verwendete Strahlung kaum streuend sind, so dass die Eindringtiefe bei der OCT vergrößert wird. Handelsübliche OCT-Geräte besitzen beispielsweise eine Bildgebungstiefe von zirka 3 mm.

Im Bereich der Augenheilkunde lassen sich mittels der OCT Informationen über den Zustand und den inneren Aufbau des Auges gewinnen. Diese Informationen können als Grundlage für die Steuerung einer nachfolgenden Behandlung des Auges herangezogen werden.

Solche Behandlungen werden mittlerweile häufig mit Lasern durchgeführt. Beispielsweise beschreibt die DE 10 2008 005 053 A1 ein Verfahren zur Korrektur von Sehfehlern an der natürlichen Augenlinse. Durch eine Laserbehandlung der Augenlinse kann auch die Altersweitsichtigkeit (Presbyopie) behandelt werden. Das menschliche Auge wird presbyop (altersweitsichtig), weil die Linse des menschlichen Auges mit zunehmendem Alter an Elastizität verliert, was zu einer Verhärtung der Linse und somit zu einem Verlust an Akkommodation Samplitude führt. Mit zunehmendem Alter kann die Abbildung nur noch über einen immer kleineren Bereich scharf auf die Netzhaut fokussiert werden. Eine Behandlung der Presbyopie wird dadurch ermöglicht, dass die Augenlinse mit einem Laser bearbeitet wird, um ihre Flexibilität wieder zu erhöhen.

In einem späteren Stadium verhärtet sich die menschliche Augenlinse üblicherweise noch weiter und trübt ein. Diese Erkrankung bezeichnet man als Katarakt (Grauer Star). Zu ihrer Behandlung wird die verhärtete Linse mit einer Ultraschallkanüle zerkleinert und aus dem Auge entfernt. Als Ersatz wird eine Kunstlinse implantiert.

Sowohl die Behandlung der Presbyopie, als auch das Zerkleinern der verhärteten Linse beim Katarakt kann durch Ultrakurzpulslaser erfolgen. Die Foki der einzelnen Laserpulse müssen dabei innerhalb der Augenlinse gezielt an vorbestimmte Stellen platziert werden, so dass sich das gewünschte Behandlungsmuster ergibt.

Von Patient zu Patient unterscheiden sich jedoch die Dimensionen der Komponenten des Auges. Erst wenn die räumliche Beziehung dieser Komponenten und ihre Ausdehnung genau bekannt ist, kann auch eine Laserbehandlung des Auges exakt durchgeführt werden.

Nachteilig an den herkömmlichen OCT-Geräten ist in diesem Zusammenhang die bereits erwähnte Begrenzung ihrer Bildgebungstiefe auf zirka 3 mm. Der für eine Laserbehandlung eines Auges relevante Bereich des Auges umfasst die Hornhaut (Kornea), den Bereich zwischen der Kornea und der Linsenvorderfläche, und die Augenlinse. Diese Komponenten des Auges haben entlang der optischen Achse die folgenden Ausdehnungen: Die Kornea hat eine Dicke von zirka 500 µm, der Abstand zwischen der Kornearückseite und der Linsenvorderfläche beträgt etwa 4 mm und die Augenlinse selbst hat eine Dicke von 4 bis 6 mm. Daraus ergibt sich, dass mit handelsüblichen OCT-Systemen weder die Augenlinse insgesamt, noch die gesamte Vorderkammer zwischen Kornearückseite und Linsenvorderfläche dargestellt werden kann. Umgekehrt sind die einzelnen Bestandteile des Auges dabei aber wesentlich dünner als die Bildgebungstiefe des OCT beziehungsweise hochgradig transparent für die verwendete Strahlung. Folglich wird der Darstellungsbereich von einigen Millimetern aufgrund der Größe der Probe häufig nicht vollständig ausgenutzt.

Die US 2007/0081166 A1 offenbart ein Handgerät zum Durchführen von OCT an einem Auge. Dieses Handgerät umfasst eine Lichtquelle, einen Detektor und einen Strahlteiler zum Aufteilen der Strahlung in einen Probenarm und einen Referenzarm.

Eine deutlich kompliziertere Interferometrie-Anordnung für wissenschaftliche Zwecke, die auch nicht mehr in einem Handgerät untergebracht werden kann, geht aus der US 2006/062260 A1 hervor.

Die US 2001/0000978 A1 beschreibt eine Vorrichtung zum Gewinnen von Daten zur dreidimensionalen Verteilung von rückgestreutem Licht aus transparenten oder halbtransparenten Objekten wie einem menschlichen Auge. Die US 2007/0206197 A1 beschreibt ein OCT-System mit der Möglichkeit zur spektral aufgelösten Mikroskopie. Aus der WO 01/38820 A1 gehen ein Verfahren und eine Vorrichtung zur Messung optischer Eigenschaften wenigstens zweier voneinander beabstandeter Bereiche in einem transparenten und/oder diffusiven Gegenstand hervor. Und die US 2008/0285043 A1 offenbart eine Vorrichtung für interferometrische Messungen einer Probe, beispielsweise eines Auges.

Weitere Interferometrie-Anordnungen sind aus der US 2006/0139656 A1 oder der WO 2009/149103 A1 bekannt.

Aufgabe der vorliegenden Erfindung ist es, mit konstruktiv möglichst einfachen Mitteln eine Anordnung und ein Verfahren zur Interferometrie zur Verfügung zu stellen, mit denen eine deutlich verbesserte Darstellung der zu untersuchenden Probe ermöglicht wird.

Diese Aufgabe wird gelöst durch eine Anordnung mit den Merkmalen des Anspruchs 1 beziehungsweise durch ein Verfahren mit den Merkmalen des Anspruchs 7. Vorteilhafte Weiterbildungen der Erfindung sind in den Unteransprüchen angegeben.

Die erfindungsgemäße (optische beziehungsweise Mess-) Anordnung zur Interferometrie zeichnet sich dadurch aus, dass in dem Strahlengang des Probenarms ein für die Strahlung teiltransparentes, einen Teil der Strahlung zum Detektor reflektierendes optisches Referenzelement angeordnet ist, hinter dem die zu untersuchende Probe positionierbar ist. Sofern die Probe nicht weiter von der reflektierenden Oberfläche des Referenzelements entfernt ist als die Kohärenzlänge der verwendeten Strahlung, kann eine interferometrische Messung mit den aus der Probe und am Referenzelement reflektierten Strahlanteilen nach Art eines Common-Path-Interferometers durchgeführt werden. Zusätzlich kann eine weitere interferometrische Messung mit dem aus der Probe reflektierten und dem den Referenzarm durchlaufenden Strahlanteil erfolgen (d.h. nach Art eines Michelson-Interferometers). Es ergeben sich also auf konstruktiv einfache Weise zwei interferometrische Pfade. Der Vorteil der Erfindung liegt darin, dass die beiden interferometrischen Messungen nicht nur gleichzeitig erfolgen können, sondern unterschiedliche Tiefen in der zu untersuchenden Probe erfassen können. Die interferometrische Messung mit dem am Referenzelement reflektierten Strahlungsanteil wird Strukturen nahe der Oberfläche der Probe erfassen (beispielsweise bis in eine Tiefe von 3 mm), während die interferometrische Messung mit dem Strahlungsanteil aus dem Referenzarm tiefere Strukturen in der Probe erfassen wird (beispielsweise in einer Tiefe von 3 bis 6 mm). Darüber hinaus bietet die Erfindung unter anderem die folgenden Vorteile:
- Die erfindungsgemäße Anordnung zur Interferometrie ist vergleichsweise einfach aufgebaut (und folglich kaum störanfällig), da sie - im Gegensatz beispielsweise zur Dual- oder Multi-Beam-OCT mit einer einzigen Strahlteilung auskommt.
- Indem die erfindungsgemäße Interferometrie-Anordnung mehrere Referenzarme vermeidet beziehungsweise den Probenarm als zweiten Referenzarm nutzt, gelangt mehr Licht auf die Probe, und das Signal-Rausch-Verhältnis verbessert sich.
- Durch den leicht messbaren (oder sogar auf Null verringerbaren) Abstand zwischen der reflektierenden Oberfläche des Referenzelements und der Probe ist die Tiefeninformation der in der Probe ermittelten Strukturen intrinsisch und exakt definiert.

Vorzugsweise ist die der Probe zugewandte Fläche des Referenzelements eben. Durch eine teilreflektierende Beschichtung und/oder durch einen Brechungsindexsprung an dieser Fläche kann erreicht werden, dass von dieser Fläche ein Teil der kohärenten Strahlung auf den Detektor reflektiert wird. Günstig ist es dabei, wenn die teilreflektierende Fläche des Referenzelements so ausgerichtet ist, dass möglichst viel reflektiertes Licht zum Detektor gelangt. Wenn diese Fläche eben ist, erleichtert dies zudem das Bestimmen des Abstandes zwischen der Referenzfläche des Referenzelements und der Probe.

Besonders günstig ist es, wenn die Probe in direktem Kontakt mit dem Referenzelement positionierbar ist. Auf diese Weise kann der Brechungsindexsprung zwischen dem Material des Referenzelements (beispielsweise Glas) und der Probe zum Reflektieren der kohärenten Strahlung ausgenutzt werden. Günstig ist diese Position auch deshalb, weil dadurch ein besonders tiefer Bereich der Probe noch innerhalb der Kohärenzlänge der optischen Strahlung liegt, gemessen von der teilreflektierenden Fläche des Referenzelements. Darüber hinaus bietet sich bei elastischen oder gel-artigen Proben (beispielsweise weichem Gewebe wie Augen, aber auch elastomeren Kunststoffen) der Vorteil, dass die Probe durch den Kontakt mit dem Referenzelement in eine definierte Form gebracht wird.

Bevorzugt ist das Referenzelement keilförmig ausgebildet. Im Unterschied zur Verwendung einer planparallelen Platte wird dadurch eine weitere interferometrische Referenzierung durch das von der Oberseite der Platte nochmals auf die Probe reflektierte Licht vermieden.

In einer vorteilhaften Variante der Erfindung ist - jeweils ausgehend vom Strahlteiler - die optische Weglänge des Referenzarms und/oder des Probenarms veränderbar. Dies ermöglicht es, Strukturen in unterschiedlichen Tiefen in der Probe interferometrisch zu erfassen. Zudem können die Messbereiche in unterschiedlichen Tiefen teilweise in Überdeckung miteinander gebracht werden, so dass die räumliche Beziehung der in einem Messbereich erfassten Strukturen zu den im anderen Messbereich erfassten Strukturen erkennbar wird. Mit einem herkömmlichen Common-Path-Interferometer ist eine unabhängige Änderung der optischen Weglänge des Referenzarms und des Probenarms nicht möglich.

Durch die erfindungsgemäße Kombination der beiden Interferometertypen können die jeweiligen Referenzflächen gegen die Probe getrennt voneinander verschoben werden. Dabei ist es besonders zweckmäßig, wenn die optischen Weglängen des Referenzarms und/oder des Probenarms so einstellbar sind, dass diese optischen Weglängen (jeweils ausgehend vom Strahlteiler) einander völlig angeglichen werden. Wenn diese Weglängen einander entsprechen, erfassen die beiden interferometrischen Pfade der erfindungsgemäßen Anordnung Strukturen in derselben Tiefe innerhalb der Probe. Dadurch können die beiden interferometrischen Pfade gegeneinander geeicht werden.

Im Probenarm ist ein Fokussierelement vorgesehen. Es sorgt dafür, die Untersuchung der Probe auf einen bestimmten Bereich zu beschränken und innerhalb dieses Bereichs die zur Verfügung stehende Lichtintensität zu erhöhen.

Besonders günstig ist es, wenn das Fokussierelement eine solche Brechkraft für die Strahlung aufweist, dass die optische Weglänge des Probenarms vom Strahlteiler bis zum Fokus des Fokussierelements maximal um die Rayleighlänge der Strahlung länger ist als die optische Weglänge des Referenzarms. Die Rayleighlänge ist diejenige Distanz entlang der optischen Achse, innerhalb derer sich die Querschnittsfläche eines Laserstrahls (ausgehend von der Strahltaille) verdoppelt. Diese Rayleighlänge z_{R} kann berechnet werden durch die Formel Z_{R} = π · w₀² / λ, wobei w₀ der Strahlradius im Fokus und λ die Wellenlänge des verwendeten Lichts ist. Wenn die durch die beiden interferometrischen Pfade der erfindungsgemäßen Anordnung darstellbaren Bereiche der Probe gemeinsam innerhalb der Rayleighlänge liegen, ergeben sich ein optimales Signal-Rausch-Verhältnis und eine optimale laterale Auflösung (da andernfalls die Lichtintensität zwischen dem einen untersuchten Bereich und dem anderen untersuchten Bereich zu stark abfällt).

Die Erfindung bezieht sich nicht nur auf eine interferometrische Anordnung, sondern auch auf ein Verfahren zur Interferometrie, das mit der vorstehend beschriebenen Anordnung durchführbar ist. Bei diesem Verfahren wird die Interferenz eines ersten, den Referenzarm durchlaufenden Strahlanteils mit einem zweiten, aus der Probe auf den Detektor gelangenden Strahlanteil gemessen. Zusätzlich wird die Interferenz eines dritten, von einem für die Strahlung teiltransparenten, im Probenarm vor der Probe angeordneten Referenzelement auf dem Detektor gelangenden Strahlanteils mit einem vierten, aus der Probe auf den Detektor gelangenden Strahlanteil gemessen. Wie bereits vorstehend erläutert wurde, können so auf konstruktiv einfache Weise und gegebenenfalls sogar gleichzeitig Informationen über Strukturen aus unterschiedlichen tiefen Bereichen der Probe gewonnen werden. Gegenüber einer herkömmlichen Interferometriemessung, beispielsweise durch ein herkömmliches OCT-Gerät, kann der erfasste Tiefenbereich beispielsweise von 3 mm auf 6 mm verdoppelt werden, bei gleichzeitigem Erhalt der Auflösung eines herkömmlichen Systems mit einem Tiefenbereich von lediglich 3 mm.

Wie vorstehend bereits eriäutert wurde, ergeben sich weitere Vorteile, wenn die Probe bei der Messung in direktem Kontakt mit dem Referenzelement positioniert ist, wenn die durch die beiden interferometrischen Pfade der Messanordnung erfassten Bereiche der Probe nicht weiter als die durch das Fokussierelement definierte Rayleighlänge der Strahlung voneinander entfernt sind und/oder wenn der Referenzarm und der Probenarm zum Kalibrieren so eingestellt werden, dass ihre optischen Weglängen gleich sind.

Durch das erfindungsgemäße Verfahren können nicht nur in-vivo-Proben untersucht werden, sondern auch ex-vivo-Proben, beispielsweise extrahierte oder künstliche Proben biologischen oder organischen Materials, Pflanzen oder geeignete Kunststoffe oder Gläser.

Im Folgenden wird ein vorteilhaftes Ausführungsbeispiel der Erfindung anhand einer Zeichnung näher erläutert. Im Einzelnen zeigen:
- Figur 1A: einen schematischen Aufbau der erfindungsgemäßen optischen Anordnung;
- Figur 1B: eine schematische Ansicht der mit der erfindungsgemäßen Anordnung gewonnenen Messdaten;
- Figur 2A: einen ersten interferometrischen Pfad der in Figur 1A gezeigten Anordnung,
- Figur 2B: eine Darstellung der in dem ersten interferometrischen Pfad gewonnenen Messdaten;
- Figur 3A: einen zweiten interferometrischen Pfad der in Figur 1A gezeigten Anordnung, und
- Figur 3B: eine Darstellung der in dem zweiten interferometrischen Pfad gewonnenen Messdaten.

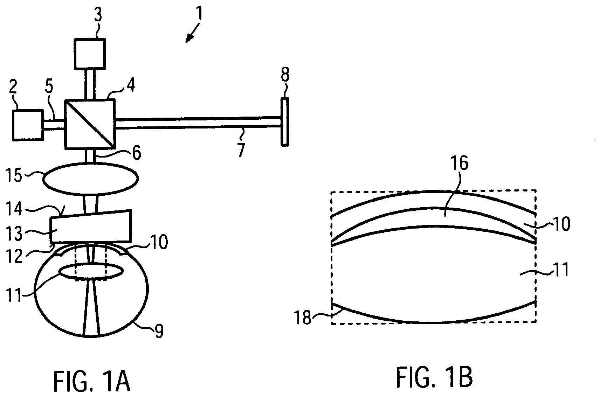

Figur 1A zeigt in schematischer Darstellung eine erfindungsgemäße (Mess-) Anordnung 1. Die Anordnung 1 verfügt über eine Lichtquelle 2 zum Erzeugen koheränter Strahlung, d.h. einen Laser. Bei dem Laser kann es sich um einen gepulsten Laser handeln, beispielsweise um einen Ultrakurzpulslaser, der ein für die FD-OCT geeignetes, breites Spektrum erzeugt.

Ein Detektor 3 ist zum Erfassen von interferometrischen Messdaten vorgesehen. Bei dem Detektor 3 kann es sich um eine CCD-Kamera handeln. Der Detektor 3 ist mit einer geeigneten Auswerteeinheit und einer Anzeigevorrichtung verbunden (nicht dargestellt).

Ein Strahlteiler 4 teilt die von der Lichtquelle 2 erzeugte, kohärente Strahlung 5 auf in einen Probenarm 6 und einen Referenzarm 7. Bei dem Strahlteiler 4 handelt es sich um den einzigen Strahlteiler innerhalb der erfindungsgemäßen Anordnung 1. In alternativen Ausführungsbeispielen können auch mehrere Strahlteiler 4 verwendet und durch optische Fasern gekoppelt werden, sodass man verschiedene Aufbauten voneinander trennen kann

Am Ende des Referenzarms 7 befindet sich ein Reflektor 8. Er ist so ausgerichtet, dass das den Referenzarm 7 durchlaufende und von ihm zurückreflektierte Licht über den Strahlteiler 4 auf den Detektor 3 gelenkt wird.

Am Ende des Probenarms 6 ist die zu untersuchende Probe 9 angeordnet, in dem dargestellten Fall ein menschliches Auge. Dieses Auge 9 verfügt über eine Hornhaut (Kornea) 10 und eine Augenlinse 11.

Ein Bereich der Vorderseite der Kornea 10 ist in unmittelbarem Kontakt mit einer ebenen Rückfläche 12 eines Referenzelements 13. Durch den Kontakt, vorzugsweise noch unterstützt durch ein Andrücken des Referenzelements 13 an die Probe 9, wirkt das Referenzelement 13 applanierend auf den mit ihm in Kontakt befindlichen Teil der Probe 9. Das Referenzelement 13 ist als optischer Keil ausgebildet, indem seine Vorderseite 14 nicht parallel zur Rückfläche 12 ausgerichtet ist.

Die Rückfläche 12 des Referenzelements 13 ist für die den Probenarm 6 durchlaufende Strahlung 5 teilreflektierend, und zwar durch eine entsprechende Beschichtung und/oder durch einen Brechungsindexsprung zwischen dem Referenzelement 13 und dem Material der Probe 9. Sie bildet dadurch die Referenzfläche des Referenzelements. Das Referenzelement 13 kann beispielsweise aus Glas bestehen.

Zwischen dem Strahlteiler 4 und dem Referenzelement 13 ist als Fokussierelement 15 eine Linse angeordnet. Die Linse 15 ist so gewählt, dass sie die Strahlung im Probenarm 6 vergleichsweise schwach fokussiert. Dadurch wird die Rayleighlänge der Strahlung relativ groß und es lassen sich über die beiden interferometrischen Pfade der Anordnung 1 Informationen aus deutlich (d.h. mehrere Millimeter) voneinander entfernten Tiefen der Probe 9 gewinnen. Liegt die Zentralwellenlänge der von der Lichtquelle 2 erzeugten Strahlung 5 beispielsweise bei etwa 700 nm, könnte der durch die Linse 15 erzeugte Strahlradius w₀ 30 µm betragen, um eine Rayleighlänge von etwa 4 mm zu erhalten. Bis zu dieser Rayleighlänge z_{R} können Zentren der die beiden durch die jeweiligen interferometrischen Pfade der Anordnung 1 erfassten Bereiche voneinander entfernt sein. Maximal dürften die beiden erfassten Bereiche sogar bis zur doppelten Rayleighlänge voneinander entfernt sein, um die beiden Messungen noch mit einem guten Signal-Rausch-Verhältnis und einer guten lateralen Auflösung durchführen zu können.

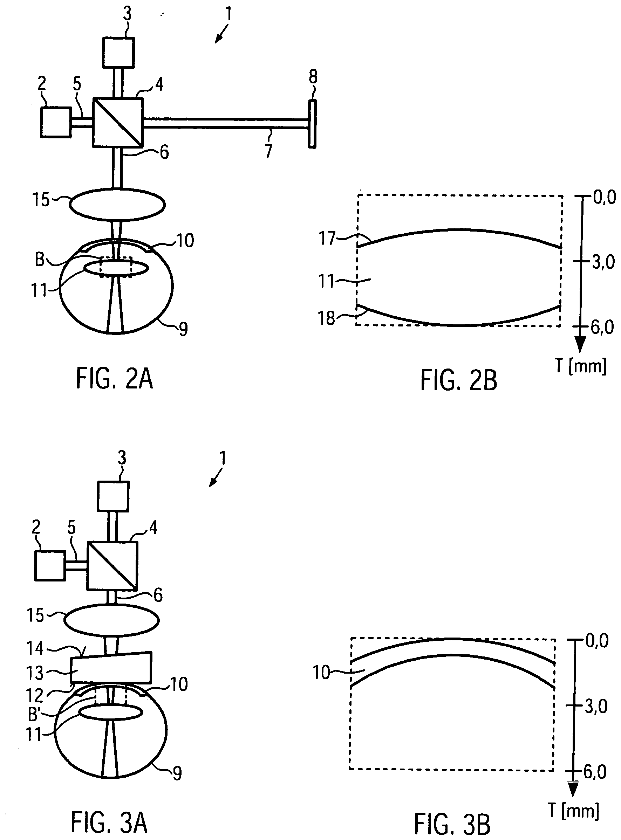

Figur 1B zeigt die mit der Anordnung 1 gewonnenen Informationen über die Strukturen in der Probe 9, im vorliegenden Fall also über die Topographie der Hornhaut 10 und der Augenlinse 11, sowie über die zwischen diesen beiden Strukturen liegende Vorderkammer 16 des Auges 9. Auf welche Weise diese Daten gewonnen werden, wird nachfolgend mit Bezug auf die Figuren 2 und 3 erläutert.

Figur 2A zeigt schematisch einen ersten interferometrischen Pfade der erfindungsgemäßen Anordnung 1. Bei diesem ersten interferometrischen Pfad spielt die Rückreflexion durch das Referenzelement 13 keine Rolle. Das Referenzelement 13 ist daher in Figur 2A ausgeblendet.

Der in Figur 2A dargestellte, erste interferometrische Pfad entspricht einem Michelson-Interferometer. Ein erster Strahlanteil der von der Lichtquelle 2 erzeugten Strahlung 5 durchläuft den Referenzarm 7 und gelangt von dort über den Strahlteiler 4 zum Detektor 3. Im Detektor 3 interferiert dieser erste Strahlanteil mit einem zweiten, aus der Probe 9 auf den Detektor 3 gelangenden Strahlanteil. Diese interferometrische Messung kann in Form einer TD-OCT erfolgen (bei der die Tiefe der in der Probe 9 erfassten Strukturen durch die optische Weglänge des Referenzarms 7 bestimmt wird), bevorzugt jedoch mittels einer FD-OCT, bei der unterschiedliche Spektralanteile zur Interferenz gebracht werden, um gleichzeitig Informationen über unterschiedliche Tiefen in der Probe 9 zu erhalten. Das Fokussierelement 15 ist dabei so gewählt und positioniert, dass in der zu untersuchenden Tiefe der Probe 9 eine ausreichende Lichtintensität zur Verfügung steht.

Der Bereich B der Probe 9, aus dem durch die interferometrische Messung Informationen gewonnen werden, ist in Figur 2A durch einen strichliert gezeichneten Kasten dargestellt. Der entsprechende Ausschnitt, der sich auch auf einer Anzeigevorrichtung des Detektors 3 ergeben wird, ist vergrößert in Figur 2B gezeigt. Zu sehen ist hier, dass durch die interferometrische Messung im ersten Pfad Informationen über die Topographie der Augenlinse 11 gewonnen werden, nämlich über die Lage und die Krümmung ihrer Vorderfläche 17 und ihrer Rückfläche 18. Diese Flächen liegen in einer Tiefe T von beispielsweise 2 bis 5 mm unter der Oberfläche des Auges 9.

Figur 3A zeigt in schematischer Darstellung einen zweiten interferometrischen Pfad der Anordnung 1. Da der Referenzarm 7 in diesem Pfad keine Rolle spielt, ist er in Figur 3A ausgeblendet. Der in Figur 3A gezeigte, zweite interferometrische Pfad entspricht einem Common-Path-Interferometer. Dabei wird ein dritter Anteil der Strahlung 5 im Probenarm 6 von der teilreflektierenden Rückfläche 12 des Referenzelements 13 auf den Detektor 3 reflektiert. Dort interferiert diese dritter Strahl am Teil mit einem vierten, aus der Probe auf den Detektor gelangenden Strahlanteil, soweit dieser vierte Strahlanteil innerhalb der Kohärenzlänge der Strahlung liegt (ausgehend von der Rückfläche 12 des Referenzelements 13). Der Bereich B' innerhalb der Probe 9, aus dem Informationen gewonnen werden, liegt somit dicht unter der Oberfläche der Probe 9. Dieser Bereich B' ist in Figur 3A mit einem strichlierten Kasten und in Figur 3B in einer Vergrößerung dargestellt. Im vorliegenden Ausführungsbeispiel wird durch den zweiten interferometrischen Pfad eine Information über die Struktur und die Krümmung der Hornhaut 10 des Auges 9 gewonnen. In Figur 3B ist deutlich zu erkennen, dass der mittlere Abschnitt der Hornhaut 10 durch die Anlage am Referenzelement 13 applaniert ist. Durch den zweiten interferometrischen Pfad lassen sich Informationen in einer Tiefe von 0 bis 3 mm aus der Probe 9 gewinnen.

Die in Figur 1A gezeigte, erfindungsgemäße Messanordnung 1 ist eine Kombination oder Überlagerung der beiden in den Figuren 2A und 3A gezeigten, interferometrischen Pfade. Dabei werden mehrere optische Komponenten gemeinsam genutzt, beispielsweise der Strahlteiler 4 und das Fokussierelement 15. Das erfindungsgemäße Verfahren sieht vor, die Messungen der beiden Pfade der interferometrischen Anordnung gleichzeitig oder nacheinander auszuführen. Indem die Messbereiche der beiden interferometrischen Pfade in unterschiedlichen Tiefen der Probe 9 liegen, kann über einen weiten Tiefenbereich der Probe 9 Information über den inneren Aufbau der Probe erhalten werden. Die Messergebnisse der Messungen in den beiden interferometrischen Pfaden können auch kombiniert werden, wie dies in Figur 1B dargestellt ist. Figur 1B zeigt die Strukturen sowohl der Augonlinse 11, als auch der Hornhaut 10 des untersuchten Auges 9. Indem übereinstimmende Strukturen aus den beiden Randbereichen der jeweiligen Messbereiche B, B' miteinander überlagert werden (beispielsweise durch eine Strukturen erkennende, bildverarbeitende Software), können die räumlichen Beziehungen der in den beiden Messbereichen B, B' ermittelten Strukturen erkannt werden. Zusätzlich oder alternativ zu einer solchen Überlagerung von übereinstimmenden strukturellen Bildmerkmalen besteht die Möglichkeit, die beiden interferometrischen Pfade gegeneinander abzugleichen. Zu diesem Zweck wird die optische Weglänge des Referenzarms 7 so eingestellt, dass sie möglichst exakt der optischen Weglänge des Probenarms 6 entspricht, d.h. dem Abstand zwischen dem Strahlteiler 4 und der teilreflektierenden Fläche 12 des Referenzelements 13.

Ausgehend von dem dargestellten Ausführungsbeispiel kann die erfindungsgemäße Anordnung 1 und das erfindungsgemäße Verfahren auf vielfache Weise abgewandelt werden. Beispielsweise ist es möglich, die Anordnung nicht als FD-OCT, sondern als TD-OCT zu betreiben.

## Patentansprüche

1. Anordnung (1) zur Interferometrie, umfassend:
- eine Lichtquelle (2) zum Erzeugen kohärenter Strahlung (5),
- einen mit einer Auswerteeinheit verbundenen Detektor (3),
- einen Strahlteiler (4) zum Aufteilen der von der Lichtquelle (2) erzeugten Strahlung (5) in einen Probenarm (6), in dem eine zu untersuchende Probe (9) positionierbar ist, und einen Referenzarm (7),
**dadurch gekennzeichnet, dass** in dem Strahlengang des Probenarms (6) ein für die Strahlung teiltransparentes, einen Teil der Strahlung zum Detektor (3) reflektierendes optisches Referenzelement (13) angeordnet ist, hinter dem die zu untersuchende Probe (9) positionierbar ist,
dass im Probenarm (6) ein Fokussierelement (15) vorhanden ist, wobei das Fokussierelement (15) eine solche Brechkraft für die Strahlung (5) aufweist, dass die optische Weglänge des Probenarms (6) vom Strahlteiler (4) bis zum Fokus des Fokussierelements (15) maximal um die Rayleighlänge der Strahlung länger ist als die optische Weglänge des Referenzarms (7),
dass ein Reflektor (8) im Referenzarm (7) und/oder das optische Referenzelement (13) im Probenarm (6) unabhängig voneinander versetzt werden können, und
dass die Anordnung konfiguriert ist zum voneinander unabhängigen Durchführen zweier verschiedener Messungen zum Gewinnen von Informationen über Strukturen aus unterschiedlichen Tiefen der Probe (9),
wobei eine erste Messung basiert auf der Interferenz eines ersten, den Referenzarm (7) durchlaufenden Strahlanteils mit einem zweiten, aus der Probe (9) auf den Detektor (3) gelangenden Strahlanteil, und
wobei eine zweite Messung basiert auf der Interferenz eines dritten, vom Referenzelement (13) reflektierten und auf den Detektor (3) gelangenden Strahlanteils mit einem vierten, aus der Probe (9) auf den Detektor (3) gelangenden Strahlanteil.

2. Anordnung nach Anspruch 1, **dadurch gekennzeichnet, dass** das Referenzelement (13) eine der Probe (9) zugewandte, ebene Fläche (12) aufweist.

3. Anordnung nach einem der vorangehenden Ansprüche, **dadurch gekennzeichnet, dass** die Probe (9) in direktem Kontakt mit dem Referenzelement (13) positionierbar ist.

4. Anordnung nach einem der vorangehenden Ansprüche, **dadurch gekennzeichnet, dass** das Referenzelement (13) keilförmig ausgebildet ist.

5. Anordnung nach einem der vorangehenden Ansprüche, **dadurch gekennzeichnet, dass** - jeweils ausgehend vom Strahlteiler - die optische Weglänge des Referenzarms (7) und/oder des Probenarms (6) veränderbar ist.

6. Anordnung nach einem der vorangehenden Ansprüche, **dadurch gekennzeichnet, dass** die optische Weglänge des Referenzarms (7) und/oder des Probenarms (6) so einstellbar ist, dass die optischen Weglängen -jeweils ausgehend vom Strahlteiler (4) - einander gleich sind.

7. Verfahren zur Interferometrie, bei dem kohärente Strahlung (5) in einen Probenarm (6) und einen Referenzarm (7) aufgeteilt wird, bei dem im Probenarm (6) eine zu untersuchende Probe (9) positioniert wird, und bei dem mittels eines mit einer Auswerteeinheit verbundenen Detektors (3) Folgendes gemessen wird:
- in einer ersten Messung: die Interferenz eines ersten, den Referenzarm (7) durchlaufenden Strahlanteils mit einem zweiten, aus der Probe (9) auf den Detektor (3) gelangenden Strahlanteil, und
- in einer zweiten Messung: die Interferenz eines dritten, von einem für die Strahlung (5) teiltransparenten, im Probenarm (6) vor der Probe (9) angeordneten Referenzelement (13) auf den Detektor (3) gelangenden Strahlanteils mit einem vierten, aus der Probe (9) auf den Detektor (3) gelangenden Strahlanteil,
wobei die erste und die zweite Messung unabhängig voneinander durchgeführt werden und zum Gewinnen von Informationen über Strukturen aus unterschiedlichen Tiefen der Probe (9) dienen,
wobei ferner die Strahlung im Probenarm (6) mittels eines Fokussierelements (15) fokussiert wird und das Fokussierelement (15) eine solche Brechkraft für die Strahlung (5) aufweist, dass die optische Weglänge des Probenarms (6) vom Strahlteiler (4) bis zum Fokus des Fokussierelements (15) maximal um die Rayleighlänge der Strahlung (5) länger ist als die optische Weglänge des Referenzarms (7),
und wobei die optische Weglänge des Referenzarmes (7) und/oder die Position des Referenzelementes (13) im Probenarm (6) unabhängig voneinander verändert werden.

8. Verfahren nach Anspruch 7, **dadurch gekennzeichnet, dass** die Probe (9) bei der Messung in direktem Kontakt mit dem Referenzelement (13) positioniert ist.

9. Verfahren nach einem der Ansprüche 7 oder 8, **dadurch gekennzeichnet, dass** der Referenzarm (7) und der Probenarm (6) zum Kalibrieren so eingestellt werden, dass ihre optischen Weglängen gleich sind.

## Claims

1. Arrangement (1) for interferometry, comprising:
- a light source (2) for generating coherent radiation (5),
- a detector connected with a processing unit,
- a beam splitter (4) for dividing the radiation (5) generated by the light source (2) into a sample arm (6) in which a sample (9) to be examined can be positioned, and a reference arm (7),
**characterized in that** an optical reference element (13), which is partially transparent to the radiation and which reflects a portion of the radiation towards the detector (3), is arranged in the beam path of the sample arm (6), behind which optical reference element (13) the sample (9) to be examined can be positioned,
**in that** a focusing element (15) is present in the sample arm (6), the focusing element (15) having such a reflective power for the radiation (5) that the optical path length of the sample arm (6) from the beam splitter (4) to the focus of the focusing element (15) is longer than the optical path length of the reference arm (7) maximally by the Rayleigh length of the radiation,
**in that** a reflector (8) in the reference arm (7) and/or the optical reference element (13) in the sample arm (6) can be offset independently of each other, and
**in that** the arrangement is configured for independently from each other conducting two different measurements for obtaining information on structures from different depths of the sample (9),
wherein a first measurement is based on the interference of a first beam portion passing the reference arm (7) with a second beam portion reaching the detector (3) from the sample (9), and
wherein a second measurement is based on the interference of a third beam portion being reflected from the reference element (13) and reaching the detector (3) with a fourth beam portion reaching the detector (3) from the sample (9).

2. Arrangement according to claim 1, **characterized in that** the reference element (13) comprises a planar surface (12) facing the sample (9).

3. Arrangement according to any of the preceding claims, **characterized in that** the sample (9) can be positioned in direct contact with the reference element (13).

4. Arrangement according to any of the preceding claims, **characterized in that** the reference element (13) is wedge-shaped.

5. Arrangement according to any of the preceding claims, **characterized in that** the optical path length of the reference arm (7) and/or of the sample arm (6) - in each case starting from the beam splitter - is variable.

6. Arrangement according to any of the preceding claims, **characterized in that** the optical path length of the reference arm (7) and/or of the sample arm (6) can be adjusted such that the optical path lengths - in each case starting from the beam splitter (4) - are identical.

7. Method for interferometry, in which coherent radiation (5) is divided into a sample arm (6) and a reference arm (7), wherein in the sample arm (6) a sample (9) to be examined is positioned, and wherein the following is measured using a detector (3) connected with a processing unit:
- in a first measurement: the interference of a first part of the radiation passing the reference arm (7) with a second part of the radiation reaching the detector (3) from the sample (9), and
- in a second measurement, the interference of a third portion of radiation reaching the detector (3) from a partially transparent reference element (13) being arranged within the sample arm (6) in front of the sample (9) with a fourth portion of radiation reaching the detector (3) from the sample (9),
wherein the first and second measurement are performed independently of each other and served to obtain information on structures from different depths of the sample (9),
wherein further the radiation in the sample arm (6) is focused by a focusing element (15), and the focusing element (15) has such a reflective power for the radiation (5) that the optical path length of the sample arm (6) from the beam splitter (4) to the focus of the focusing element (15) is at maximum longer than the optical path length of the reference arm (7) by the Rayleigh length of the radiation (5),
and wherein the optical path length of the reference arm (7) and/or the position of the reference element (13) in the sample arm (6) are varied independently of each other.

8. Method according to claim 7, **characterized in that** the sample (9) is positioned during the measurement in direct contact with the reference element (13).

9. Method according to any of claims 7 or 8, **characterized in that** the reference arm (7) and the sample arm (6) are adjusted for calibration such that the optical path lengths are identical.

## Revendications

1. Appareil (1) pour interférométrie, comprenant :
- une source de lumière (2) pour générer un rayonnement cohérent (5),
- un détecteur (3) connecté à une unité d'évaluation,
- un séparateur de faisceau (4) pour diviser le rayonnement (5) généré par la source de lumière (2) en un bras d'échantillon (6) dans lequel un échantillon (9) à examiner peut être positionné, et un bras de référence (7)
**caractérisé en ce qu'**un élément de référence optique (13) est disposé dans le trajet du faisceau du bras d'échantillon (6), qui est partiellement transparent au rayonnement et réfléchit une partie du rayonnement vers le détecteur (3), derrière lequel l'échantillon (9) à examiner peut être positionné,
**en ce qu'**un élément de focalisation (15) est présent dans le bras d'échantillon (6), l'élément de focalisation (15) ayant un pouvoir de réfraction pour le rayonnement (5) tel que la longueur du trajet optique du bras d'échantillon (6) depuis le séparateur de faisceau (4) jusqu'au foyer de l'élément de focalisation (15) est au maximum supérieure à la longueur du trajet optique du bras de référence (7) de la longueur Ra-yleigh du rayonnement,
**en ce qu'**un réflecteur (8) dans le bras de référence (7) et/ou l'élément optique de référence (13) dans le bras d'échantillonnage (6) peuvent être déplacés indépendamment l'un de l'autre, et
en ce sens que l'appareil est configuré pour effectuer deux mesures différentes indépendamment l'une de l'autre afin d'obtenir des informations sur les structures à différentes profondeurs de l'échantillon (9),
dans lequel une première mesure est basée sur l'interférence d'une première composante de faisceau passant à travers le bras de référence (7) avec une seconde composante de faisceau passant de l'échantillon (9) au détecteur (3), et dans laquelle une deuxième mesure est basée sur l'interférence d'une troisième partie de faisceau réfléchie par l'élément de référence (13) et atteignant le détecteur (3) avec une quatrième partie de faisceau atteignant le détecteur (3) à partir de l'échantillon (9).

2. Appareil selon la revendication 1, **caractérisé en ce que** l'élément de référence (13) présente une surface plane (12) faisant face à l'échantillon (9).

3. Appareil selon l'une des revendications précédentes, **caractérisé en ce que** l'échantillon (9) peut être positionné en contact direct avec l'élément de référence (13).

4. Appareil selon l'une des revendications précédentes, **caractérisé en ce que** l'élément de référence (13) est en forme de coin.

5. Appareil selon l'une des revendications précédentes, **caractérisé en ce que** - en partant chaque fois du séparateur de faisceau - la longueur du trajet optique du bras de référence (7) et/ou du bras d'échantillonnage (6) peut être modifiée.

6. Appareil selon l'une des revendications précédentes, **caractérisé en ce que** la longueur du trajet optique du bras de référence (7) et/ou du bras d'échantillonnage (6) est réglable de telle sorte que les longueurs des trajets optiques - en partant chaque fois du séparateur de faisceau (4) - sont égales entre elles.

7. Procédé d'interférométrie, dans lequel le rayonnement cohérent (5) est divisé en un bras d'échantillon (6) et un bras de référence (7), dans lequel un échantillon (9) à examiner est positionné dans le bras d'échantillon (6), et dans lequel ce qui suit est mesuré au moyen d'un détecteur (3) relié à une unité d'évaluation :
- dans une première mesure : l'interférence d'une première composante du faisceau traversant le bras de référence (7) avec une seconde composante du faisceau passant de l'échantillon (9) au détecteur (3), et
- dans une deuxième mesure : l'interférence d'une troisième composante de faisceau atteignant le détecteur (3) à partir d'un élément de référence (13) qui est partiellement transparent au rayonnement (5) et est disposé dans le bras d'échantillon (6) devant l'échantillon (9) avec une quatrième composante de faisceau atteignant le détecteur (3) à partir de l'échantillon (9),
où la première et la deuxième mesure sont effectuées indépendamment l'une de l'autre et servent à obtenir des informations sur les structures à différentes profondeurs de l'échantillon (9),
dans lequel, en outre, le rayonnement dans le bras d'échantillon (6) est focalisé au moyen d'un élément de focalisation (15) et l'élément de focalisation (15) a un pouvoir de réfraction tel pour le rayonnement (5) que la longueur du chemin optique du bras d'échantillon (6) depuis le séparateur de faisceau (4) jusqu'au foyer de l'élément de focalisation (15) est au plus plus longue de la longueur de Rayleigh du rayonnement (5) que la longueur du chemin optique du bras de référence (7),
et dans lequel la longueur du trajet optique du bras de référence (7) et/ou la position de l'élément de référence (13) dans le bras d'échantillonnage (6) sont modifiées indépendamment l'une de l'autre.

8. Procédé selon la revendication 7, **caractérisé en ce que** l'échantillon (9) est placé en contact direct avec l'élément de référence (13) pendant la mesure.

9. Procédé selon l'une des revendications 7 ou 8, **caractérisé en ce que** le bras de référence (7) et le bras d'échantillonnage (6) sont ajustés pour l'étalonnage de sorte que leurs longueurs de chemin optique soient égales.
